Europäisches Patentamt

European Patent Office

Office européen des brevets

(19)

(11) Publication number: **0 222 493**

**A1**

(12)

# EUROPEAN PATENT APPLICATION

(21) Application number: 86307595.8

(22) Date of filing: 02.10.86

(51) Int. Cl.⁴: **C12N 15/00** , **C07H 21/04** , **C12P 19/34** , **A01H 1/00**

The applicant has filed a statement in accordance with Rule 28 (4) EPC (issue of a sample only to an expert).

(30) Priority: 04.10.85 US 784837

(43) Date of publication of application: 20.05.87 Bulletin 87/21

(84) Designated Contracting States: AT BE CH DE ES FR GB GR IT LI LU NL SE

(71) Applicant: **LUBRIZOL GENETICS INC. 3375 Mitchell Lane Boulder Colorado 80301-2244(US)**

(72) Inventor: **Gelvin, Stanton B. 5251 Moores Bay Road West·Lafayette Indiana 47906(US)**

(74) Representative: **Fisher, Adrian John et al Carpmaels & Ransford 43 Bloomsbury Square London WC1A 2RA(GB)**

(54) **TR-based sub-TI plasmids.**

(57) Sub-Ti plasmids based on $T_R$ -DNA from an octopine-type Ti plasmid are disclosed. A selectable marker functional in eukaryotes and prokaryotes is also exemplified, as are vectors and methods useful in efforts to transform plants.

Figure 1

## T$_R$-BASED SUB-TI PLASMIDS

FIELD

The present invention is in the fields of genetics engineering and husbandry, and especially provides vectors useful for plant transformation and means for promotion of transcript termination in plants.

BACKGROUND

Following are publications which disclose background information closely related to the present invention. These publications are discussed in greater depth in the Background section indicated. R. F. Barker et al. (1983) Plant Mol. Biol. 2:335-350, disclose the complete sequence of the T-DNA from the octopine type plasmid pTi15955; homologous published sequences of other Ti plasmid genes are referenced therein. Sub-Ti plasmids based on T$_R$-DNA and having an ORF24 promoter-based selectable marker expressible in both plants and bacteria are disclosed by S. B. Gelvin et al. (1985) Mol. Gen. Genet. 199:240-248.

Overview of Agrobacterium

Virulent strains of the gram-negative genus Agrobacterium harbor large plasmids known as Ti (tumor-inducing) plasmids (pTi) in A. tumefaciens and Ri (root-inducing) plasmids (pRi) in A . rhizogenes, often classified by the opine which they caused to be catabolized. Ti and Ri plasmids both contain DNA sequences, known as T-DNA (transferred-DNA), which in tumors are found to be integrated into the genome of the host plant. Several T-DNA genes are under control of T-DNA promoters which resemble canonical eukaryotic promoters in structure. The Ti plasmid also carries genes outside the T-DNA region.

General reviews of Agrobacterium-caused disease include those by D. J. Merlo (1982), Adv. Plant Pathol. 1:139-178; L. W. Ream and M. P. Gordon (1982), Science 218:854-859; M. W. Bevan and M. -D. Chilton (1982), Ann. Rev. Genet. 16:357-384; G. Kahl and J. Schell (1982) Molecular Biology of Plant Tumors; K. A. Barton and M.-D. Chilton (1983) Meth. Enzymol. 101:527-539; A. Depicker et al. (1983) in Genetics Engineering of Plants: an Agricultural Perspective, eds: T. Kosuge et al., pp. 143-176; A. Caplan et al. (1983) Science 222:815-821; T. C. Hall et al., European Patent application 126,546; and A. N. Binns - (1984) Oxford Surveys Plant Mol. Cell Biol. 1:130-160. A number of more specialized reviews can be found in A. Pühler, ed. (1983) Molecular Genetics of the Bacteria-Plant Interaction. R. A. Schilperoort (1984) in Efficiency in Plant Breeding (Proc. 10th Congr. Eur. Assoc. Res. Plant Breeding), eds: W. Lange et al., pp. 251-285, discusses the Agrobacterium-based plant transformation in the context of the art of plant genetic engineering and plant improvement.

Infection of Plant Tissues

Plant cells can be transformed by Agrobacterium by a number of methods known to the art. For a review of recent work, se K. Syono (1984) Oxford Surveys Plant Mol. Cell Biol. 1:217-219. In the present invention, any method will suffice as long as the gene is stably transmitted through mitosis and meiosis.

The infection of plant tissue by Agrobacterium is a simple technique well known to those skilled in th ¬ art. Typically after being wounded, a plant is inoculated with a suspension of tumor-inducing bacteria. Alternatively, tissue pieces are inoculated, e.g. leaf disks (R. B. Horsch et al. (1985) Science 227:1229-1231) or inverted stem segments (K. A. Barton et al. (1983) Cell 32:1033-1043). After induction, the tumors can be placed in tissue culture on media lacking phytohormones usually included for culture of untransformed plant tissue. Traditional inoculation and culture techniques may be modified for use of disarmed T-DNA vectors incapable of hormone independent growth (e.g. see P. Zambryski et al. (1984) in Genetic Engineering, Priniciples, and Methods, 6, eds. A. Hollaender and J. Setlow).

Agrobacterium is also capable of infecting isolated cells, cells grown in culture, callus cells, and isolated protoplasts (e.g. R. B. Horsch and R. T. Fraley (1983) in Advances in Gene Technology: Molecular Genetics of Plants and Animals (Miami Winter Symposium 20), eds.: K. Downey et al., p. 576; R.T. Fraley et al. - (1984) Plant Mol. Biol. 3:371-378; R. T. Fraley and R. B. Horsch (1983) in Genetic Engineering of Plants: an Agricultural Perspective, eds.: T. Kosuge et al ., pp. 177-194; A. Muller et al. (1983) Biochem. Biophys. Res. Comm. 123:458-462). The transformation frequency of inoculated callus pieces can be increased by addition of an opine or opine precursors (L.M. Cello and W. L. Olsen, U.S. Patent 4,459,355).

The host range of crown gall pathogenesis may be influenced by T-DNA-encoded functions such as onc genes (A. Hoekema et al. (1984) J.Bacteriol.158:383-385; A. Hoekema et al. (1984) EMBO J. 3:3043-3047; W. C. Buchholz and M. F. Thomasshow (1984) 160:327-332). R. L. Ausich, European Patent Application 108,580, reports transfer of T-DNA from A. tumefaciens to green algal cells, and expression therein of octopine synthase and Tn5 kanamycin resistance genes. G. M. S. Hooykaas-van Slogteren et al. - (1984) Nature 311:763-764, and J.-P. Hernalsteens et al. (1984) EMBO J. 3 :3039-3041, have demonstrated transformation of monocot cells by Agrobacterium without the customary tumorigenesis.


Regeneration of Plants

Differentiated plant tissues with normal morphology have been obtained from crown gall tumors. For example, Ti plasmid mutants have been used to create tumors which proliferated shoots that formed self-fertile flowers. The resultant seeds germinated into plants which contained T-DNA and made opines. The rootless phenotype of tissue transformed by tms$^+$ tmr$^-$ T-DNA can be partly overcome by washing of the rooting area and can be bypassed by grafting onto a normal stock (A. Wöstemeyer et al. (1984) Mol. Gen. Genet. 194:500-507). Similar experiments with a tmr $^-$ T-DNA showed that full-length T-DNA could be transmitted through meiosis to progeny and that in those progeny nopaline genes could be expressed, though at variable levels (K. A. Barton et al. (1983) Cell 32:1003-1043).

Genes involved in opine anabolism were capable of passing through meiosis, though the plants were male sterile if the T-DNA was not disarmed. Seemingly unaltered T-DNA and functional foreign genes can be inherited in a dominant, closely linked, Mendelian fashion (e.g. see R. B. Horsch et al. (1984) Science 223:496-498; D. Tepfer (1984) Cell 37 :959-967; M. De Block et al. (1984) EMBO J. 3:1681-1689; A. Wöstemeyer et al. (1984) Mol. Gen. Genet. 194500-507).

The epigenetic state of the plant cells initially transformed can affect regeneration potential (G. M. S. van Slogteren et al . (1983) Plant Mol. Biol. 2-321-333).

Regeneration after transformation by A. rhizogenesis discussed by M.-D. Chilton et al. (1982) Nature 295:432-434; C. David et al. (1984) Biotechnol. 2:73-76; and P. Constantino et al. (1984) J. Mol. Appl. Genet. 2:465-470.


Genes on the Transformation Inducing Plasmids

The complete sequence of the T-DNA of an octopine-type plasmid found in ATCC 15955, pTi15955, has been reported (R. F. Barker et al. (1983) Plant Molec. Biol. 2:335-350) as has the T $_L$ region of pTiAch5 (J. Gielen et al . (1984) EMBO J. 3:835-846). Published T-DNA genes do not contain introns. Sequences resembling canonical eukaryotic promoter elements and polyadenylation sites can be recognized.

A number of genes have been identified within the T-DNA of transformation inducing plasmids. Several octopine plasmid T-DNA transcripts have been mapped (e.g. L. Willmitzer et al. (1982) EMBO J. 1:139-146; S. J. Karcher et al. (1984) Mol. Gen. Genet. 194:159-165) and some functions have been assigned. Some of these regions, in particular those encoding tmr and tms, can also be transcribed in prokaryotic cells (G. Schröder et al. (1983) EMBO J. 2:403-409). Ti plasmids having mutations in the genes tms, tmr, tml, and ocs respectively incite tumorous calli of Nicotiana tabacum which generate shoots, proliferate roots, are larger than normal, and do not synthesize octopine; all by ocs are onc (oncogenicity) genes. In other hosts, mutants of these genes can induce different phenotypes (see Bevan and Chilton (1982) Ann. Rev. Genet., supra). Mutations in T-DNA genes do not affect insertion of T-DNA into the plant genome (J. Leemans et al. (1982) EMBO J. 1:147-152; L. W. Ream et al. (1983) Proc. Natl. Acad. Sci. USA 80:1660-1664).

Octopine Ti plasmids carry an ocs gene which encodes octopine synthase (lysopine dehydrogenase). C. Koncz et al. (1983) EMBO J. 2:1597-1603, provides a functional analysis of ocs. P. Dhaese et al. (1983) EMBO J. 2:419-426, reported the utilization of various polyadenylation sites by "transcript 7" (ORF3 of Barker et al., supra) and ocs. The presence of the enzyme octopine synthase within a tissue can protect that tissue from the toxic effect of various amino acid analogs (G. A. Dahl and J. Tempe (1983) Theor. Appl. Genet. 66:233-239; M. G. Koziel et al. (1984) J. Mol. Appl. Genet. 2:549-562).

Nopaline Ti plasmids encode the nopaline synthase gene (nos) (sequenced by A. Depicker et al. - (1982) J. Mol. Appl. Genet. 1:561-573). C. H. Shaw et al. (1984) Nucl. Acids Res. 12:7831-7846, provides a functional analysis of nos. Genes equivalent to tms and tmr have been identified on a nopaline-type plasmid and a number of transcripts have been mapped (L. Willmitzer et al. (1983) Cell 32:1045-1056).

Transformation inducing plasmid genes outside of the T-DNA region include the vir genes, which when mutated result in an avirulent Ti plasmid. Several vir genes have been accurately mapped and have been found to be located in regions conserved among various Ti plasmids (V. N. Iyer et al. (1982) Mol. Gen. Genet. 188:418-424). The vir genes function in trans, being capable of causing the transformation of plant cells with T-DNA of a different plasmid type and physically located on another plasmid (e.g. A. J. de Framond et al. (1983) Biotechnol. 1:262-269; A. Hoekema et al. (1983) Nature 303:179-180; J. Hille et al. - (1984) J. Bacteriol. 158:754-756; A. Hoekema et al . (1984) J. Bacteriol. 158:383-385; G. An et al. (1985) EMBO J. 4:277-284); such arrangements are known as binary systems. Chilton et al . (1983) 15th Miami Winter Symp., described a "micro-Ti" plasmid made by resectioning the mini-Ti of de Framond et al., supra (see European patent application 126,546 for a description). G. A. Dahl et al., U.S. Patent application ser. no. 532,280, See EP-A-0140556 and A. Hoeing (1985) Ph.D. Thesis, Rijksuniversiteit Leiden, The Netherlands, disclose micro-Ti plasmids carrying ocs genes constructed from the $T_L$ region of the octopine-type plasmid pTi15955. M. Bevan (1984) Nucl. Acids Res. 12:8711-8712, discloses a kanamycin-resistant micro-Ti. T-DNA need not be on a plasmid to transform a plant cell; chromosomally located T-DNA is functional - (A. Hoekema et al. (1980) EMBO J. 3:2485-2490). T-DNA has direct repeats of about 25 base pairs associated with the borders, i.e. with the T-DNA/plant DNA junctions, which may be involved in either transfer from Agrobacterium or integration into the host genome. The four pTi15955 borders have been designated A, B, C, and D by Barker et al., supra. Ti plasmid-determined characteristics have been reviewed by Merlo, supra (see especially Table II therein), and Ream and Gordon, supra.

Transformation Inducing Plasmid DNA

Some transformation inducing plasmids, including octopine-type Ti plasmids, integrate two separate T-DNAs, $T_L$ and $T_R$-DNA, left and right T-DNAs, respectively. The copy numbers of pTi $T_R$ and $T_L$ can vary in different tumor lines. A core of octopine T-DNA is highly homologous to nopaline T-DNA, is required for tumor maintenance, is found in $T_L$, is generally present in one copy per cell, and codes for tumor morphology genes. (Tumor genes of some Ti plasmids may be integrated in two segments (M. F. Thomashow etal. (1980) Cell 19:729-739; W. G. Buchholz and M. F. Thomashow 91984) J. Bacteriol. 160:319-326).) $T_R$ can be totally dispensed with but can be found in a high copy number. Though T-DNA is occasionally deleted after integration in the plant genome, it is generally stable. Tumors containing a mixture of cells which differ in T-DNA organization are sometimes the result of multiple transformation events. Nopaline T-DNA is integrated into the host genome as an uninterrupted segment.

The exact location relative to the border repeats of T-DNA/flanking plant DNA junctions varies and need not be within a border repeat. Virulence is not always eliminated after deletion of one of either of the usual nopaline T-DNA border sequences (compare H. Joos et al. (1983) Cell 32:1057-1067 with K. Wang et al. - (1984) Cell 38:455-462 and C. H. Shaw et al. (1984) Nucl. Acids Res. 12 :6031-6041, concerning the right border). The orientation of the right nopaline border can be reversed without total loss of functionality, and a single border sequence is capable of transforming closely-linked sequences (M. De Block et al. (1984) EMBO J. 3:1681-1689). A synthetic 25 bp nopaline right border repeat is functional (Wang et al., supra).

## Shuttle Vectors

"Shuttle vectors", developed by G. B. Ruvkun and F. M. Ausubel (1981) Nature 289:85-88, provide means for inserting foreign genetic material into large DNA molecules. Shuttle vectors include copies of recipient genome DNA sequences into which the foreign genetic material is inserted. They can be introduced into the ultimate recipient cells by well known methods including the tri-parental mating technique (Ruvkin and Ausubel, supra), direct transfer of a self-mobilizable vector in a biparental mating, direct uptake of exogenous DNA by Agrobacterium cells ("transformation"), spheroplast fusion of Agrobacterium with another bacterial cell, or uptake of liposome-encapsulated DNA.

After a shuttle vector is introduced into the recipient cell, possible events include a double cross-over with one recombinational event on either side of the marker (homogenotization). Phenotypically dominant traits may be introduced by single cross-over events (cointegration) (A. Caplan et al. (1983) Science 222:815-821; R. B. Horsch et al. (1984) Science 223:496-498); one must guard against deletion of the resulting tandem duplication. Shuttle vectors have proved useful in manipulation of Agrobacterium plasmids.

"Suicide vectors" (e.g. R. Simon et al. (1983) Biotechnol. 1:784-791), are shuttle vectors having replicons not independently maintainable within the recipient cell. Use of suicide vectors to transfer DNA sequences into a Ti plasmid has been reported (e.g. E. Van Haute et al. (1983) EMBO J. 2:411-417; L. Comai et al. (1983) Plasmid 10:21-30; P. Zambryski et al. (1983) EMBO J. 2:2143-2150; P. Zambryski et al. (1984) inGenetic Engineering, Principles, and Methods, 6, eds: A. Hollaender and J. Setlow; P. Zahn et al. - (1984) Mol. Gen. Genet. 194:188-194; Caplan et al., supra; C. H. Shaw et al. (1983) Gene 28:315-330).

## Foreign Gene Expression

the nopaline synthase gene was found to be fully functional when inserted into octopine T-DNA and transformed into plant tissue (C. L. Fink (1982) M.S. thesis, University of Wisconsin-Madison). A gene encoding bean phaseolin has been transferred into and expressed in sunflower tumors. Transcription started and stopped at the correct positions, and introns were posttranscriptionally processed properly (N. Murai et al. (1983) Science 222:476-482). An intronless gene for the endosperm protein zein, from the monocot Zea mays, is transcribed though not translated in sunflower callus (M. A. Matzke et al. (1984) EMBO J. 3:1525-1531). Expression of a pea RuBP-Case small subunit gene is light-regulated in transformed petunia cells; the pea small subunit protein produced is correctly processed and sequestered within chloroplasts (R. Broglie et al. (1984) Science 224:838-843). A chimeric small subunit/NPT2 protein is also translocated into the chloroplast (G. Van den Broeck et al. (1985) Nature 313:358-363). That gene's promoter confers light-inducible expression in callus to bacterial structural genes (L. Herrera-Estrella et al. (1984) Nature 310 :115-120; d. Facciotti et al. (1985) Biotechnol. 3:241-246).

The nos promoter can drive expression of drug resistance structural genes useful for selection of transformed plant cells. M. W. Bevan et al . (1983) Nature 304:184-187; R. T. Fraley et al. (1983) Proc. Natl. Acad. Sci. USA 80:4803-4807; and L. Herrera-Estrella et al. (1983) EMBO J. 2:987-995, have inserted bacterial kanamycin resistance structural gene (neomycin phosphotransferase II, NPT2), or kan, from Tn5 gene behind (i.e. under control of) the nopaline synthase promoter. The constructions were used to transform plant cells which in culture were resistant to kanamycin and its analogs such as neomycin and G418. Promoters for octopine $T_L$ genes ORF24 and ORF25 can also drive kan structural gene expression (J. Velten etal. (1984) EMBO J. 3:2723-2730). Herrera-Estrella et al., supra, reported a similar construction, in which a methotrexate resistance gene (dihydrofolate reductase) from Tn7 was placed behind the nos promoter. Transformed cells were resistant to methotrexate. The NPT2 gene under control of a cauliflower mosaic virus promoter is expressed in plant cells transformed by DNA directly taken up into cells (J. Paszkowski et al. (1984) EMBO J. 3:2817-2722; R. C. Gardner et al. (1984) Plant Mol. Biol. Reporter 2:3-8) or by T-DNA (M. G. Koziel et al. (1984) J. Mol. Appl. Genet. 2:549-562. Furthermore, L. Herrera-Estrella et al. (1983) Nature 303:209-213, have obtained expression in plant cells of enzymatic activity of octopine synthase and chloramphenicol acetyltransferase by placing their structural genes under control of nos promoters. G. Helmer et al. (1984) Biotechnol. 2:250-527, have created a fusion gene useful as a screenable marker having the promoter and 5'-end of the structural gene of nos fused to E. coli @-galactosidase (lacZ) sequences.

Murai et al., supra, reported fusion of the promoter and the 5'-end of the ocs structural gene to a phaseolin structural gene. The encoded fusion protein was produced under control of the T-DNA promoter. Phaseolin-derived introns were posttranscriptionally processed properly.

## SUMMARY OF THE INVENTION

One object of this invention is to provide means for expression of structural genes within plant cells wherein said genes are foreign to said cells. Another object is to provide disarmed plant transformation vectors compatible with the growth of morphologically normal plants. Yet another object is to provide specialized plant tissues and plants having within them proteins encoded by foreign structural genes and, in cases where the protein is an enzyme, having or lacking metabolites or chemicals which respectively are not or are otherwise found in the cells in which the genes is inserted. Towards these ends, sub-Ti plasmids containing $T_R$-DNA are provided. Further objects and advantages will become evident from the following description.

The invention disclosed herein provides sub-Ti plasmids based on octopine pTi $T_R$-DNA, that is having borders C and D while lacking border A. In the preferred embodiments these sub-Ti plasmids lack onc genes and lack DNA outside of octopine-type pTi BamHI fragment 2. The use of sub-Ti plasmids containing the direct border repeats involved in incorporation of the T-DNA into the plant genome can have the following useful results: 1. onc genes can be deleted resulting in greater success of plant regeneration from transformed tissue cultures or protoplasts. 2. Plant cells can be transformed by $T_L$, $T_R$, or both $T_L$ and $T_R$. Since multiple copies of $T_R$ can be found in a transformed plant genome and it is actively transcribed, deletion of all or part of $T_L$ can result in a high level of expression of foreign genes in the absence of various onc genes. 3. Sub-Ti plasmids are relatively small, thereby facilitating or elimina ting many manipulations otherwise required during the process of plant transformation.

The invention also provides a plant comprising a genetically modified plant cell having a foreign structural gene introduced by such a sub-Ti plasmid and expressed therein under control, as exemplified herein, of a T-DNA-derived, plant-expressible transcription controlling sequence. Further, the invention provides plant tissue comprising a plant cell having a genome comprising a foreign structural gene inserted in such orientation and spacing with respect to T-DNA-derived plant expressible transcriptional control sequences as to be expressible in the plant cell under control of those sequences. Also provided are novel strains of bacteria containing and replicating T-DNA, the T-DNA being modified to contain an inserted foreign structural gene in such orientation and spacing with respect to a T-DNA-derived promoter and a T-DNA-derived transcript terminator so as to be expressible in a plant under control of said promoter region and terminator. Additionally, the invention provides novel vectors useful in efforts to transform plants. These vectors have the ability to replicate in bacteria and comprising $T_R$-DNA. They further comprise a foreign structural gene inserted within $T_R$-DNA contained within the vector, in such manner as to be expressible in a plant cell or a bacterium under control of the T-DNA-derived promoter region and T-DNA transcript terminator. Furthermore, strains of bacteria harboring said vectors are disclosed.

The present invention comprises foreign structural genes under control of a promoter, said promoter/gene combination being inserted into a cell by means of sub-Ti plasmids based on octopine-type Ti plasmid $T_R$-DNA. More specifically, in its preferred embodiment the invention disclosed herein further comprises expression in plant cells of foreign structural genes under control of a certain T-DNA-derived, plant-expressible promoter, derived from DNA flanking ORF24, after introduction via T-DNA, that is to say, by inserting the foreign structural gene into T-DNA under control of the ORF24 promoter and ahead of an ORF25 or ORF26 polyadenylation site and introducing the T-DNA containing the insert into a plant cell using known means. Once plant cells expressing the foreign structural gene are obtained, plant tissues and whole plants can be regen erated therefrom using methods and techniques well known in the art. The regenerated plants are then reproduced by conventional means and the introduced genus can be transferred to other strains and cultivars by conventional plant breeding techniques. The invention in principle applies to any introduction of a foreign structural gene into any plant species into which DNA can be introduced.

The invention is useful for genetically modifying bacteria, plant cells, plant tissues, and whole plants by inserting useful foreign structural genes from other species, organisms, or strains. Such useful structural genes include, but are not limited to, genes conveying identifiable phenotypes such as the following: improved tolerance to extremes of heat or cold; improved tolerance to anaerobic conditions (e.g. water-logging), drought, or osmotic stress; improved resistance or tolerance to insect (e.g. insecticidal toxins such as the Bacillus thuringiensis crystal protein), arachnid, nematode, or epiphyte pests and fungal, bacterial, or viral diseases; the production of enzymes or secondary metabolites not normally found in said tissues or plants; improved nutritional (e.g. lectins and storage proteins such as zein or phaseolin), flavor (e.g. sweet proteins such as thaumatin), or processing properties when used for fiber or human or animal food; changed morphological traits or developmental patterns (e.g. leaf hairs which protect the plant from insects, coloring which is aesthetically pleasing, changed plant growth habits, dwarf plants, reduced time needed for the plants to reach maturity, expression of a gene in a tissue or at a time that gene is not usually expressed, and the like); male sterility; improved photosynthetic efficiency (including lowered photorespiration); improved nitrogen fixation; improved uptake of nutrients; improved tolerance to herbicides (e.g. glyphosate or triazines); increased crop yield; improved competition with other plants; genetic markers novel to the genetically modified cell; and the like. Genetic markers can be used to improve germplasm identification by the presence of one or more characteristic nucleic acid sequences, proteins, gene products, or phenotypes however identified. Genetic markers can distinguish a genetically modified plant, plant cell, or bacterial cell of the present invention from plants, plant cells, or bacteria which are not so modified, to facilitate transfer of a genetically linked or cotransformed artificially introduced DNA sequence or phenotype by other (e.g. sexual) means to other genotypes, or to facilitate identification of plants protected by patents or by plant variety protection certificates. Resistance (or tolerance) in cell or tissue culture to selective agents (i.e. selectable markers) and markers that are readily identified during screening (e.g. screenable markers such as distinctive cell-surface antigens or enzymes, like @-galactosidase, that are readily recognized visually) are also useful genetic markers. The invention is examplified by placing a structural gene, encoding neomycin phosphotransferase II (NPT2) from Tn5 and providing a phenotype of resistance to the effects of kanamycin and its analogs (a kan gene), under control of a promoter region which in nature controls expression of the ORF24 and ahead of a polyadenylation site found between ORF25 and ORF26. The promoter/kan polyadenylation site combination can be used to detect and select cells transformed by the combination. Any DNA sequences linked to the combination may be selected for, both in eukaryotes and prokaryotes, and cells transformed by linked DNA sequences may therefore be identified, as will be understood by those in the art. Other uses of the invention, exploiting the properties of other structural genes introduced into various plant species will be readily apparent to those skilled in the art.

## DESCRIPTION OF THE DRAWINGS

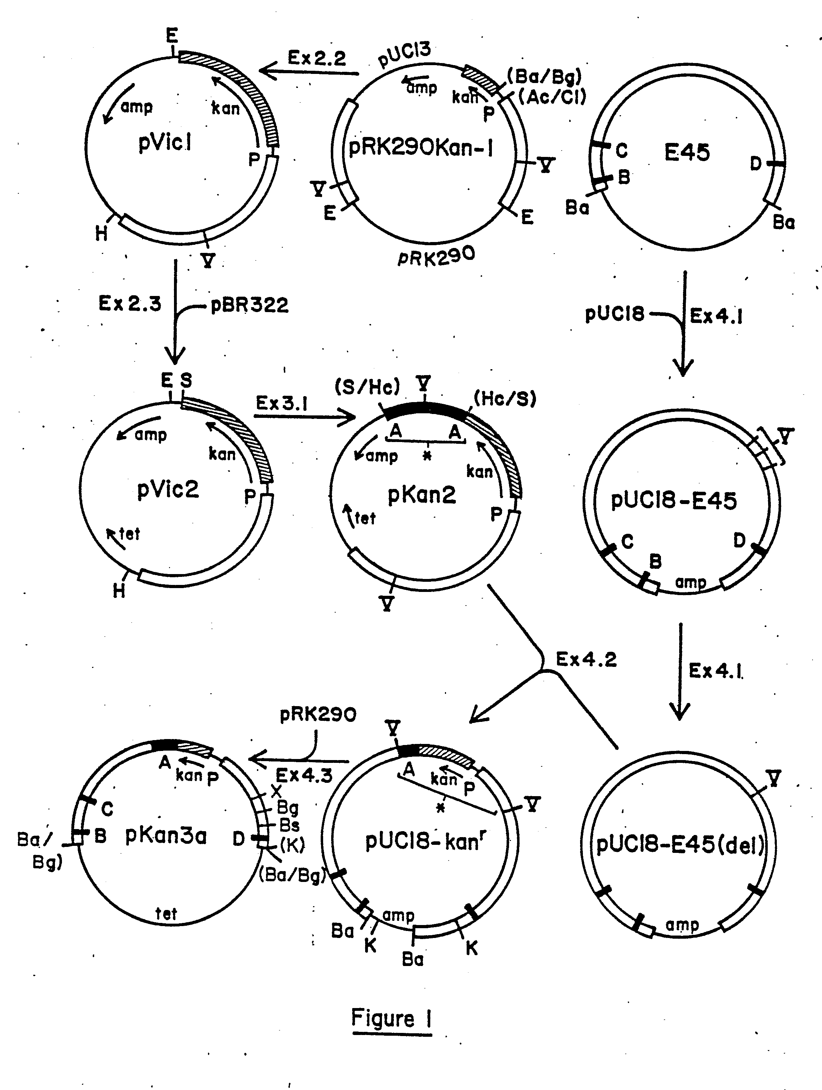

Figure 1 is a schematic diagram, not drawn to scale, of DNA manipulations utilized in Examples 2, 3, and 4. Thin curved lines represent vector (pRK290, PbR322, pUC13, and pUC18) sequences; open boxes represent T-DNA; solid black boxes represent a transcript terminator segment; and hatched boxes represent Tn5 sequences. Sites susceptible to the action of a restriction enzyme are indicated as follows: Ac is AccI; Ba, BamHI; Bg, BglII; Bs, BstEII; Cl, ClaI; E, EcoRI; H, HindIII; Hc, HincII; K, KpnI; S, SmaI; V, EcoRV; and X represents XorII. A site or combination of two sites that is no longer susceptible to any enzyme is indicated by the presence of parenthesis around the name of the enzyme(s). "P" represents the ORF24 promoter; "A" represents a transcript terminator (polyadenylation site); amp, tet, and kan are antibiotic resistances; and "B", "C", and "D" indicate the corresponding octopine T-DNA border repeat sequences; and DNA segments that may be present in either orientation are denoted by an asterisk, "*". The extent and polarity of a structural gene is indicated by an arrow. Names of plasmids are within the circular representations of the plasmids. "Ex" refers to the Example which describes a particular manipulation.

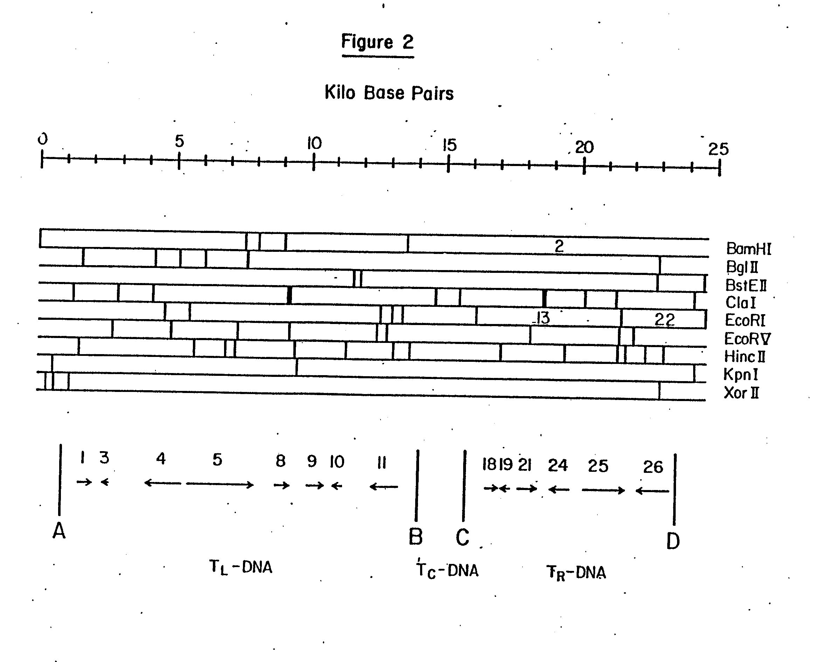

Figure 2 is a map of octopine-type T-DNA. A map of positions of restriction and sites and fragments relevant to the Examples is presented below a distance scale. Numbers within the open boxes of the map denote particular restriction fragments. Positions and orientations of eukaryotic open reading frames are indicated by arrows. Positions of border repeats are indicated by vertical bars.

## DETAILED DESCRIPTION OF THE INVENTION

The following definitions are provided, in order to remove ambiguities to the extent or scope of their usage in the specification and claims.

Promoter : Refers to sequences at the 5'-end of a structural gene involved in initiation of transcription. In nature the T-DNA promoter region sequences exemplified herein causes transcription in crown gall tumors of the ORF24. Eukaryotic promoter sequences are commonly recognized by the presence of DNA sequences homologous to the canonical form 5'...TATAA...3' about 10-30 base pairs (bp) 5'-to the location of the 5'-end of the mRNA (cap site). About 30 bp 5'0to the TATAA another canonical sequence is often found which is recognized by the presence of DNA sequences homologous to the canonical form 5'...CCAAT...3'. Translational initiation is usually most efficient at the first 5'...AUG...3' 3'-from the cap site.

Transcript Terminator: Refers herein to any nucleic acid sequence capable of determining the position of the 3'-end of a transcript. The transcript terminator DNA segment may itself be a composite of segments derived from a plurality of sources, naturally occurring or synthetic, prokaryotic, or eukaryotic, and may be from a genomic DNA or and mRNA-derived cDNA. Transcript termination sites include polyadenylation sites and sites determining the 3'-end of ribosomal RNAs (rRNAs), transfer RNAs, (tRNAs), and nonpolyadenylated messenger RNAs (mRNAs) (e.g. histone mRNA, P. A. Krieg and D. A. Melton (1984) Nature 308:203-206).

Polyadenylation Site: Refers herein to a nucleic acid sequence correlated with polyadenylation of mRNA in eukaryotes, i.e. after transcriptional termination polyadenylic acid "tails" are added to the 3'-end of mRNA precursors. Polyadenylation sites are commonly recognized by the presence of homology to the canonical form 5'...AATAAA...3', although variations of distance 5' to the 3'-end of the transcript, partial "read-thru", and multiple tandem canonical sequences are not uncommon. DNA sequences between 20 and 35 bp downstream from the transcripts 3'-end seem to be necessary (M. A. McDevitt et al. (1984) Cell 37:993-999). It should be recognized that a canonical "polyadenylation site" may actually determine the location of the 3'-end of the mRNA and not polyadenylation per se (N. Proudfoot (1984) Nature 307:412-413).

Transcription controlling sequences: Refers to a promoter/polyadenylation site combination flanking a structural gene.

Foreign structural gene: As used herein includes that portion of a gene comprising a DNA segment coding for a RNA, protein, polypeptide or portion thereof, possibly including a translational start codon, foreign to, i.e. not naturally found in, $T_R$-DNA. A foreign structural gene may be derived in whole or in part from prokaryotic DNA, eukaryotic DNA, episomal DNA, plasmid DNA, plastid DNA, genomic DNA, cDNA, viral DNA, viral cDNA, chemically synthesized DNA, or the like. It is further contemplated that a foreign structural gene may contain one or more modifications in either the coding segments or untranslated regions which could affect the biological activity or chemical structure of the expression product, the rate of expression or the manner of expression control. Such modifications include, but are not limited to, mutations, insertions, deletions, and substitutions of one or more nucleotides, and "silent" modifications that do not alter the chemical structure of the expression product but which affect intercellular localization, transport, excretion or stability of the expression product. The structural gene may constitute an uninterrupted coding sequence or it may include one or more introns, bounded by the appropriate plant functional splice junctions, which may be obtained from synthetic or a naturally occurring source. The structural gene may be a composite of segments derived from a plurality of sources, naturally occurring or synthetic, coding for a composite protein, the composite protein being foreign to the cell into which the gene is introduced and expressed or being derived in part from a foreign protein. The foreign structural gene may be a fusion protein, and in particular, may be fused to all or part of a $T_R$-DNA structural gene derived from that which the transcriptional conrol sequences were derived.

Extraneous coding DNA: Refers to a DNA segment which, when expressed in a plant cell, conveys a useful or identifiable phenotype. Such phenotypes include those suggested for foreign structural genes in the Summary. An extraneous coding DNA usually will include a DNA sequence, which is to be expressed as an RNA, flanked by a promoter and a transcript terminator. In many cases the expressed RNA will be a translatable messenger RNA. As exemplified herein, the extraneous coding is a maize (Zea mays) seed storage protein (zein) gene.

Replicon: Refers herein to a fundamental unit of replication comprising all the genetic elements sufficient to confer autonomous replication in a bacterial cell, e.g. Agrobacterium. Individual replicons differ in the extent to which they are functional in different host cell species. Many of the replicons commonly employed in plasmids for genetic engineering are functional only in bacteria of the E. coli group. Others are able to replicate in a wide range of gram-negative bacterial hosts, including Agrobacterium. A replicon will always include an origon of replication. A replicon will also include genes for functions necessary for DNA replication in a particular host cell which are not supplied by that host cell.

Plant tissue: Includes differentiated and undifferentiated tissues of plants including but not limited to roots, shoots, pollen, seeds, tumor tissue, such as crown galls, and various forms of aggregations of plant cells in culture, such as embryos and calluses. The plant tissue may be in plant or in organ, tissue, or cell culture.

Plant cell: As used herein includes plant cells in planta and plant cells and protoplasts in culture.

Bacterial cell : As used herein includes bacteria in culture, including but not limited to biologically pure cultures, and dispersed in the environment.

Positions in, open reading frames (ORFs) of, and border sequences of octopine T-DNA are defined as numbered or designated by R. F. Barker et al. (1983) Plant Mol. Biol. 2:335-350.

Production of a genetically modified cell transformed by a T $_R$-DNA-derived sub-Ti plasmid and expressing a T-DNA promoter/foreign structural gene/T-DNA transcript terminator combination combines the specific teachings of the present disclosure with a variety of techniques and expedients known in the art. In most instances, alternative expedients exist for each stage of the overall process. Alternative expedients include, but are not limited to the particular vir-gene-containing helper strain to be used as a sub-Ti plasmid helper, the particular T-DNA promoter, foreign structural gene, or T-DNA transcript terminator used, and the plant species to be modified and the desired regeneration strategy, all of which present alternative process steps which those of ordinary skill are able to select and use to achieve a desired result. For instance, although the starting point for obtaining a $T_R$-DNA-derived sub-Ti plasmid is exemplified in the present application by T-DNA isolated from pTiA6, DNA sequences of other homologous Ti plasmids might be substituted as long as appropriate modifications are made to the promoter region isolation and manipulation procedures. (Often, pTi15955 may be used as an alternative source of octopine-type T-DNA sequences.) Sub-Ti plasmids other than those derived from the pTiA6 may be discovered or constructed. Homologous genes may be identified by those of ordinary skill in the art by the ability of homologous nucleic acids to cross-hybridize under conditions of appropriate stringency or by comparison of nucleic acid or protein sequences, as is well understood in the art. It will be understood that there may be minor sequence variations within gene sequences utilized or disclosed in the present application. These variations may be determined by standard techniques to enable those of ordinary skill in the art to manipulate and bring into utility the promoter regions of such homologous genes. As strains harboring novel helper plasmids are developed for use in binary plant transformation systems, those of ordinary skill in the art will be able to select among those alternate process steps to achieve a desired result. The fundamental aspects of the invention are the nature of the .$T_L$-DNA-derived sub-Ti plasmid and its use to transform plant cells with a promoter/structural gene/transcript terminator combination or with a derivative of such a combination. Other aspects include the nature and structure of the foreign structural gene and the promoter controlling structural gene expression in plant cells. The remaining steps of the preferred embodiment for obtaining a genetically modified plant include inserting the promoter/structural gene/transcript terminator combination into $T_R$-DNA-derived sub-Ti plasmid, transferring the sub-Ti plasmid to an Agrobacterium helper cell and then transforming a plant cell wherein the $T_R$ -DNA becomes stably integrated as part of the plant cell genome, techniques for in vitro culture and eventual regeneration into whole plants, which may include steps for selecting and detecting transformed plant cells and steps of transferring the introduced T-DNA from the originally transformed strain into commercially acceptable cultivars, and monitoring expression in transformed plants.

A principal feature of the present invention in its preferred embodiment is the construction of a T-DNA derivative having a T-DNA-derived promoter and a T-DNA-derived transcript terminator combined with foreign structural gene, as these terms have been defined, supra. The structural gene must be inserted in correct position and orientation with respect to both the promoter and the transcript terminator. It is known that eukaryotic promoters and transcript terminators promote and terminate transcription in one direction only along the DNA. The terminator region of DNA is said to lie "downstream" or alternatively "behind" or "3'-to" the transcribed structural gene. Similarly, the promoter is "upstream", "ahead of", or "5'-to" the structural gene. Therefore, to be functional, the correct position of promoter or the terminator must be respectively "upstream" or "downstream" from the foreign structural gene. Orientation refers to the directionality of the structural gene. That portion of a structural gene which ultimately codes for the amino terminus of the foreign protein is termed the 5'-end of the structural gene, while that end which codes for amino acids near the carboxyl end of the protein is termed the 3'-end of the structural gene. Correct orientation of the foreign structural gene is with the 5'-end thereof proximal to the promoter and the 3'-end thereof proximal to the transcript terminator.

Another principal feature of the present invention in its preferred embodiments is use of plasmids comprising $T_R$-DNA or modified $T_R$-DNA, into which a promoter structural gene/transcript terminator combination is inserted, said plasmids being capable of independent replication in an <u>Agrobacterium</u> strain. As reviewed in the Background, border-delimited T-DNA of such plasmids can be transferred from an <u>Agrobacterium</u> strain to a plant cell provided the <u>Agrobacterium</u> strain contains certain <u>trans</u>-acting genes whose function is to promote the transfer of T-DNA to a plant cell. Borders are polar sequences, being nonselfcomplementary. Pairs of borders can function together when present in parallel orientation, i.e. when having homologous sequences on the same DNA strand. Comparison of published border sequences gives the consensus sequence 5'TGGAGGATAT $\begin{smallmatrix} T & C & A & G & T \\ A & G & G & A & G \end{smallmatrix}$ GCTAA $\begin{smallmatrix} A & T \\ T & C \end{smallmatrix}$ 3', the 5'-end and the 3'-end being respectively towards the left and right ends of both nopaline and octopine T-DNA, as conventionally mapped in the literature (e.g. as in Fig. 2). It is known in the art that the borders can be sufficient for transfer function but that sequences which flank the borders can affect transformation efficiency. Plasmids that contain T-DNA bounded by borders and which are able to replicate independently in an <u>Agrobacterium</u> strain are herein termed sub-Ti plasmids. Sub-Ti plasmids may have borders derived from Ti plasmids, Ri plasmids, or synthetic DNA. A spectrum of variations is possible in which the sub-Ti plasmids differ in the amount of T-DNA they contain. One end of the spectrum retains all of the T-DNA from a transformation inducing plasmid, and is termed herein mini-Ti plasmid. At the other end of the spectrum, all but an amount of DNA surrounding the T-DNA borders is deleted, the remaining portions being the minimum necessary for the sub-Ti plasmid to be transferrable and integratable in the host cell. Such plasmids are termed herein micro-Ti. Sub-Ti plasmids are advantageous in that they are small and relatively easy to manipulate directly, eliminating the need to transfer the gene to T-DNA from a shuttle vector by homologous recombination. After the desired structural gene has been inserted, they can easily be introduced directly into an <u>A</u>. <u>tumefaciens</u> or <u>A</u>. <u>rhizogenes</u> cell containing the <u>trans</u>-acting <u>vir</u> genes that promote T-DNA transfer. Introduction into an <u>Agrobacterium</u> strain is conveniently accomplished either by transformation of the <u>Agrobacterium</u> strain or by conjugal transfer from a donor bacterial cell, the techniques for which are well known to those of ordinary skill. The present invention includes sub-Ti plasmids based on $T_R$-DNA, that is having borders C and D and lacking border A and possibly border B. $T_R$-DNA-derived sub-Ti plasmids are advantageous in that they usually lack all <u>onc</u> genes and are therefore disarmed. Example 6 discloses such sub-Ti plasmids, generally based on $T_R$, and discusses some more detailed considerations.

As will be apparent to those of ordinary skill in the art, the promoter/foreign structural gene/transcript terminator combination may be placed between any restriction sites convenient for removing the combination from the plasmid it is carried on and convenient for insertion into the plant transformation or shuttle vector of choice. Location of a combination insertion site within a plant transformation vector is not critical as long as the transfer function of sequences immediately surrounding the T-DNA borders are not disrupted, since in prior art studies these regions appear to be essential for insertion of the modified T-DNA into the plant genome. Also, the combination should not disrupt the function of any selectable or screenable markers needed for identification of transformed plant cells. The T-DNA into which the combination is inserted is obtained from any of the transformation inducing plasmids, and the combination is inserted by standard techniques well known to those skilled in the art. The orientation of the combination with respect to the direction of transcription and translation of endogenous T-DNA or vector genes is not critical, either of the two possible orientations is functional. Differences in rates of expression in plants may be observed when a given combination is inserted at different locations within T-DNA, possibly because of such factors as DNA methylation and chromatin structure.

Following the strategy just described, the modified T-DNA can be transferred to plant cells by any technique known in the art, e.g. by direct infection of plants, by infection of leaf disks, or by cocultivation of plant cells with the novel <u>Agrobacterium</u> strain containing a foreign structural gene incorporated within T-DNA (see Background). Transformed cells must be selected or screened to distinguish them from untransformed cells. Selection is most readily accomplished by providing a selectable marker incorporated into the T-DNA (see Background). As exemplified herein, the promoter/structural ge..e/transcript terminator is a selectable marker, a ORF24 promoter/NPT2 structural gene/ORF25 or ORF26 polyadenylation site combination, suitable for selection of transformed plant tissues. Screening methods well known to those skilled in the art include, but are not limited to specific hybridization to characteristic nucleic acid sequences, or immunological assays. Additionally, a phenotype of any expressed gene located between the T-DNA borders can be used to identity transformed tissue.

Regeneration of transformed cells and tissues is accomplished by resort to known techniques. An object of the regeneration step is to obtain a whole plant that grows and reproduces normally but which retains integrated T-DNA. The techniques of regeneration vary somewhat according to principles known in the art, depending upon the origin of the T-DNA, the nature of any modifications thereto and the species of the transformed plant. Sub-Ti plasmids based on octopine-type $T_R$-DNA lack all onc genes, are totally disarmed, and result in transformed tissues which may be regenerated by protocols directly derived from protocols developed for untransformed tissues. The transformed tissue regeneration protocols generally will not need undue experimentation for adapt from untransformed tissue regeneration protocols, any changes resulting primarily from plant tissue infection steps and not from presence of the $T_R$-DNA vector in the transformed tissue.

The genotype of the plant tissue transformed is often chosen for the ease with which its cells can be grown and regenerated in in vitro culture and for susceptibility to the selective agent to be used. Should a cultivar of agronomic interest be unsuitable for these manipulations, a more amenable variety is first transformed. After regeneration, the newly introduced foreign structural gene/transcript terminator/$T_R$-DNA-derived sub-Ti plasmid $T_R$-DNA combination may be readily transferred to the desired agronomic cultivar by techniques well known to those skilled in the arts of plant breeding and plant genetics. Sexual crosses of transformed plants with the agronomic cultivars yield initial hybrids. These hybrids can then be back-crossed with plants of the desired genetic background. Progeny are continuously screened and/or selected for the con tinued presence of integrated foreign DNA or for a new phenotype resulting from expression of genes carried by the inserted foreign DNA. In this manner, after a number of rounds of back-crossing and selection, plants can be produced having a genotype essentially identical to the agronomically desired parents with the addition of inserted foreign DNA sequences.


EXAMPLES


The following Examples are presented for the purpose of illustrating specific embodiments within the scope of the present invention without limiting the scope; the scope being defined by the Claims. Numerous variations will be readily apparent to those of ordinary skill in the art.

The Examples utilize many techniques well known and accessible to those skilled in the arts of molecular biology and manipulation of transformation inducing plasmids and Agrobacterium; such methods are fully described in one or more of the cited references if not described in detail herein. Enzymes are obtained from commercial sources and are used according to the vendor's recommendations or other variations known to the art. Reagents, buffers and culture conditions are also known .to those in the art. Reference works containing such standard techniques include the following: R. Wu, ed. (1979) Meth. Enzymol. 68, R. Wu et al., eds. (1983) Meth. Enzymol. 100 and 101, L. Grossman and K. Moldave, eds. - (1980) Meth. Enzymol. 65, J. H. Miller (1972) Experiments in Molecular Genetics, R. Davis et al. (1980) Advanced Bacterial Genetics, R. F. Schleif and P. C. Wensink (1982) Practical Methods in Molecular Biology, and T. Maniatis et al. (1982) Molecular Cloning. Additionally, R. F. Lathe et al. (1983) Genet. Engin. 4:1-56, make useful comments on DNA manipulations.

Textual use of the name of a restriction endonuclease in isolation, e.g. "BclI", refers to use of that enzyme in an enzymatic digestion, except in a diagram where it can refer to the site of a sequence susceptible to action of that enzyme, e.g. a restriction site. In the text, restriction sites are indicated by the additional use of the word "site", e.g. "BclI site". The additional use of the word "fragment", e.g. "BclI fragment", indicates a linear double-stranded DNA molecule having ends generated by action of the named enzyme (e.g. a restriction fragment). A phrase such as "BclI/SmaI fragment" indicates that the restriction fragment was generated by the action of two different enzymes, here BclI and SmaI, the two ends resulting from the action of different enzymes. Note that the ends will have the characteristics of being "sticky" (i.e. having a single-stranded protrusion capable of base-pairing with a complementary single-stranded oligonucleotide) or "blunt" and that the specificity of a sticky-end will be determined by the specificity of the enzyme which produces it.

Positions and open reading frames (ORFs) of octopine-type T-DNA are as defined for pTi15955, which has a T-DNA essentially identical to that of pTiA6, by R. F. Barker et al. (9183) Plant Mol. Biol. 2:335-350, and are diagramed in Fig. 2.

Generally, plasmids, and only plasmids, are prefaced with a "p" e.g., pTi15955 or pUC13, an exception being the plasmid E45. Strain designations parenthetically indicate a plasmid harbored within, e.g., A. tumefaciens (pTi15955) or E . coli JM83 (pUC13). The following strains are on deposit:

E. coli K12 (pRK290Kan-1) NRRL B-15736

A. tumefaciens (pTi15955) ATCC 15955

Other plasmids and strains are widely available and accessible to those in the art; for example, pUC13 and pUC18 are commercially available, being listed, respectively, as items 27-4954-01 and 27-4949-01 in the 1984 Pharmacia Molecular Biologicals catalog (Pharmacia, Inc., 800 Centennial Ave., Piscataway, NJ 08854 USA).

Example 1

This Example discloses specific materials and methods.

1.1 Culture conditions

$\underline{E}$. $\underline{coli}$ strains harboring recombinant plasmids were grown in L broth supplemented with 0.2% casamino acids. Antibiotic concentrations used were for $\underline{E}$. $\underline{coli}$ : ampicillin, 50-100TMg/ml; tetracycline, 10TMg/ml; kanamycin, 20TMg/ml; and for $\underline{A}$. $\underline{tumefaciens}$: carbenicillin, 100TMg/ml; tetra cycline, 5TMg/ml; kanamycin, 100TMg/ml; rifampicin, 10TMg/ml; gentamycin, 100TMg/ml.

1.2 Construction of recombinant DNA plasmids

$\underline{BamHI}$ fragment 2 (Figure 1) was cloned from the $\underline{Agrobacterium}$ $\underline{tumefaciens}$ plasmid pTiB$_6$806 (grown in strain A277) into pBR322 using standard procedures well known to those of ordinary skill in the art. All restriction endonuclease reactions were performed as suggested by the suppliers (Bethesda Research Laboratories (BRL), P.L. Biochemicals, or New England Biolabs). T4 DNA ligase was purchased from BRL. Recombinant plasmids were isolated from $\underline{E}$. $\underline{coli}$ using a cleared lysate procedure (D. G. Blair $\underline{et}$ $\underline{al}$. (1972) Proc. Natl. Acad. Sci. USA $\underline{69}$:2518-2522) or an alkaline lysis procedure (N. C. Birnboim and J. Doly (1979) Nucleic. Acids Res. $\underline{7}$:1513-1523).

1.3 Triparental matings

Triparental matings were generally accomplished as described below; other variations known to those skilled in the art are also acceptable. pRK290 and pRK2013 were disclosed by G. Ditta $\underline{et}$ $\underline{al}$. (1980) Proc. Natl. Acad. Sci. USA $\underline{77}$:7347-7357, and pPHIJI by P. R. Hirsh (1978) Thesis, Univ. E. Anglia. $\underline{E}$. $\underline{coli}$ RR1 (pRK290-based-shuttle-vector) or $\underline{E}$. $\underline{coli}$ K802 (pRK290-based-shuttle-vector) was mated with $\underline{E}$. $\underline{coli}$ RR1 - (pRK2013) and A348, a transformation-inducing plasmid harboring $\underline{A}$. $\underline{tumefaciens}$ strain resistant to rifampicin. After pRK2013 was transferred to the shuttle vector carrying strain, pRK2013 mobilized the pRK290-based shuttle vector for transfer to the $\underline{Agrobacterium}$. Growth on a minimal medium incapable of supporting the growth of $\underline{E}$. $\underline{coli}$, AB glucose, containing both rifampicin and the drug to which the shuttle vector is resistant, often either kanamycin or carbenicillin (an ampicillin analog), resulted in the selection of $\underline{Agrobacterium}$ cells containing pRK290-based shuttle vector sequences. A mating of these cells with $\underline{E}$. $\underline{coli}$ (pPH1J1), strain 2104, resulted in the transfer of pPH1j1 to the $\underline{Agrobacterium}$ cells. pPH1J1 and pRK290-based shuttle vectors cannot coexist for long in the same cell. Growth on gentamycin and kanamycin select for cells which have Ti plasmids that have undergone single-or double-homologous recombination events - (cointergration or homogenotization, respectively) with the shuttle vector and now carry the desired construction. The concentrations of antibiotics used for selection were as described in Example 1.1. $\underline{E}$. $\underline{coli}$ strains were usually grown at 37°C in L-broth supplemented with 0.2% casamino acids, and $\underline{A}$. $\underline{tumefaciens}$ strains at 30°C in YEP medium.

Example 2

This Example teaches manipulations preparatory to combination of a DNA fragment bearing two transcript terminators with a promoter/structural gene combination.

2.1 pRK290Kan-1

Briefly, pRK290Kan-1, described by S. B. Gelvin etal. (1985) Mol. Gen. Genet. 199:240-248, was constructed as follows: Recombinant DNA plasmid having pTiA6 T-DNA between EcoRI sites at positions 16,202 and 21,631 (EcoRI fragment 13, Fig. 2) inserted into the EcoRI site of pRK290 was digested with ClaI and religated to itself, thereby deleting T-DNA between ClaI sites at positions 18,890 and 20,128. A 1 kbp SmaI/Bg1II fragment bearing a Tn5 kanamycin-resistance (kan) structural gene (sequenced by E. Beck et al. (1982) Gene 19:327-336), encoding the enzyme neomycin phosphotransferase II (NPT2), was ligated between the pUC13 (J. Messing (1983) Meth. Enzymol. 101:20-78) polylinker BamHI and SmaI sites. The pUC13/kan combinations was then cleaved at the polylinker AccI site and inserted into the unique ClaI site of the Cla I-deleted pRK290/fragment 13 combination, thereby substituting the pUC13/kan combination for T-DNA between the positions, 18,890 and 20,128 ClaI sites. This plasmid, pRK290Kan-1 (Fig. 1), is harbored by a deposited strain, NRRL B-15736 (E. coli K12 RR1 (pRK290Kan-1).

The kan structural gene of pRK290Kan-1 is expressible in plant cells under control of the ORF24 promoter and in bacterial cells. ORF24 transcript terminators are lacking from pRK290Kan-1. pRK290Kan-1, being homologous to T-DNA on both sides of the ORF24 structural gene, is suitable for integration into octopine-type Ti plasmids by double homologous recombination after direct transformation of A. tumefaciens cells and selection for carbenicillin resistance. After homogenotization, the T-DNA does not have a functional ORF24 gene and does not cause transformed plant cells to make the opines mannopine or agropine. After cointegration, the construction confirs carbenicillin resistance to bacteria and in plant cells allows agropine and mannopine synthesis. pRK290Kan-1 has been transformed into A. tumefaciens A348 - (pTiA6). Inoculation of plant with homogenotized transformants results in octopine$^+$ agropine$^-$ mannopine$^-$ kan$^r$ transformed plant tissue.

2.2 Deletion of pRK290 from pRK290Kan-1

pRK290-based plasmids are fairly large (more than 20 kbp) and are therefore often difficult to handle while doing recombinant DNA manipulations. The construction of plasmids which replicate via the pUC13 replicon is described below and diagrammed schematically in Fig. 1.

pRK290Kan-1 DNA was digested to completion with EcoRV, ligated to itself, thereby deleting pRK290, T-DNA between the positions 16,202 and 18,207 EcoRI and EcoRV sites, and T-DNA between the positions 21,522 and 21,631 EcoRV and EcoRI sites. The ligation mixture was then transformed into E. coli K12 JM83 (J. Messing (1979) Recomb. DNA Tech. Bull 2(2):43-48, NIH Publ. No. 79-99), a strain recommended for selection for presence of the ampicillin-resistance gene present in pUC13. Plasmids were isolated from ampicillin-resistant, tetracycline-sensitive transformants and were characterized by restriction analysis and a colony was identified which contained a plasmid designated pVicl, having sequences for pUC13, kan , and two T-DNA EcoRV/ClaI fragments (positions 18,027 to 18,890 and 20,128 to 21,522).

2.3 Construction of a mobilizable vector

The pUC series of plasmids (e.g. pUC13-based plasmids) cannot be mobilized for conjugal transfer from E. coli to A. tumefaciens by pRK2013 and must therefore be directly transformed into the recipient Agrobacterium cells. However, as pBR322-based plasmids can be mobilized by pRK2013 but do not replicate in Agrobacterium, such a plasmid is a useful suicide vector for the transfer of the ORF24 promoter/kan selectable marker into octopine-type Ti plasmids.

pVicl and pBR322 DNA, each digested by HindIII and EcoRI, were mixed, ligated together, and transformed into RRI. Plasmid DNA isolated from ampicillin resistant transformants were characterized by restriction mapping and a colony was identified which harbored a plasmid, designated pVic2, having a copy of pBR322 substituted for the pUC13 sequences of pVicl. pVic2 is a mobilizable suicide vector capable of cointegration or homogenotization into $T_R$.

Example 3

This Example describes ligation of a T-DNA transcript terminator to the 3'-end of the kan structural gene.

The transcript terminators of pTiA6 and pTi15955 ORFs 25 and 26 are both located within a DNA segment bounded by HincII sites at positions 21,727 and 22,440, as defined by Barker et al., supra. This 713 bp HincII fragment carries polyadenylation sites of two antiparallel genes, ORFs 25 and 26. When this HincII fragment, or any DNA segment having two anti-parallel polyadenylation sites, is placed behind a structural gene, one or the other polyadenylation sites will be functional for promoting transcript termination in a plant cell. This HincII fragment is a subfragment of octopine pTi EcoRI fragment 22.

A pBR322 clone of EcoRI fragment 22 was digested with HincII. The resulting fragments were mixed with and blunt-end ligated to SmaI-linearized pVic2 DNA. Ampicillin-resistant RR1 transformants were characterized by restriction mapping and a colony was identified which harbored a plasmid, designated pKan2, having an about 713 bp HincII fragment 3'-to the ORF24 promoter/kan combination. The NPT2-encoding transcript is terminated by either the ORF25 or the ORF26 polyadenylation site; characterization as to which transcript terminator was being used was not necessary.

Some alternative blunt-ended pTi15955-derived segments carrying antiparallel transcript terminator, the restriction enzyme(s) which generate them, and their sizes are as follows:

| ORFs | Enzyme(s) | Size (bp) |
| --- | --- | --- |
| 9/10 | HaeI | 1034 |
| 9/10 | HaeIII | 915 |
| 9/10 | NaeI/SmaI | 807 |
| 21/24 | AluI | 755 |
| 21/24 | ThaI | 960 |
| 25/26 | HaeI | 1163 |
| 25/26 | PvuII/StuI | 1036 |

Nonblunt-ended segments can be obtained and either used directly or have sticky-ends converted to blunt ends by methods well known to the art; e.g. a 1.11 kbp BbvI/ClaI fragment carries terminators of pTi15955 T-DNA ORFs 1 and 3.

Example 4

This Example teaches the construction of sub-Ti plasmids which include all of T$_R$. T$_R$ carries none of the genes which cause a phenotype in transformed cells of hormone-independent growth. Also note that when ocs is available to function as a selectable marker on some of the plasmids discussed herein, the need to use the ORF24 to drive expression of a selectable marker (e.g. kan) is eliminated.

4.1 Preparation of T-DNA that carries borders

Plasmid E45 is a pBR322 clone of octopine pTi BamHI fragment 2, which in pTi15955 extends rightward from position T 13,774. BamHI fragment 2 carries T$_R$, T$_C$, and the right end of T$_L$ genes, and includes borders B, C, and D, as defined by Barker et al., supra. pBR322 has an EcoRV site which would be inconvenient in manipulations described below, while pUC18 has none. E45 DNA was dephosphorylated after digestion with BamHI and then mixed with and ligated to BamHI-linearized pUC18 DNA (J. Norrander et al. (1983) Gene 26:101-106). Plasmid DNAs isolated from ampicillin-resistant, tetracycline-sensitive E. coli transformants were screened by restriction analysis and a colony was identified which harbored a plasmid, designated pUC18-E45, having octopine T-DNA BamHI fragment 2 inserted into the BamHI site of pUC18.

pUC18-E45 DNA was digested with EcoRV, ligated to itself, thereby recreating an EcoRV site at the resulting suture. The ligation mixture was then transformed into E. coli. Restriction mapping of plasmid DNAs from ampicillin-resistant transformants led to the identification of a colony harboring a plasmid, designated pUC18-E45(del) deleted for T-DNA between the positions 18,027 and 22,041 EcoRV sites.

4.2 Insertion of the kan/terminator combination between borders

EcoRV-digested pKan2 DNA was mixed with and ligated to EcoRV-linearized pUC18-E45(del) DNA. After transformation into E. coli , plasmid DNAs from transformants resistant to ampicillin and kanamycin were characterized by restriction site mapping. A colony was identified which harbored a pUC18-derived plasmid, designated pUC18-kan$^r$. pUC18-kan$^r$ has a pKan2-derived fragment inserted into the EcoRI suture of pUC18-E45(del). This pKan2-derived fragment itself is made up of a ClaI/EcoRV T-DNA fragment (positions 2,0128 to 21,522) bearing the ORF24 promoter, a small pUC13-derived linker sequence, a Tn5-derived NPTII structural gene, and a polyadenylation site-bearing T-DNA fragment extending from the position 22,041 EcoRV site to an HincII site at either position 21,727 to 22,440. pUC18-kan$^r$ has a ColE1 origin of replication and is incapable of being independently maintained in Agrobacterium .

4.3 Construction of a sub-Ti plasmid

pUC18-kan$^r$was digested with KpnI, which cleaves within the pUC18 polylinker and at the T-DNA position 24,337 KpnI site, and the resultant sticky-ends were removed with T4 DNA polymerase. The resultant 9.2 kbp linearized, blunt-ended molecules were mixed with and ligated to commercially available BamHI linkers. After excess linkers were removed by BamHI digestion, the pUC18-kan$^r$ DNA fragments were mixed with and ligated to BglII-linearized pRK290 DNA. Plasmid isolated from E. coli transformants resistant to both ampicillin and tetracycline were restriction mapped and a colony was identified with a harbored plasmid, designated pKan3a, having pUC18-kan$^r$-derived T-DNA inserted into pRK290. pKan3a is a micro-Ti plasmid maintainable in both E. coli and Agrobacterium and having borders B, C, and D, a plant-expressible kanamycin gene being between borders C and D, and having at least three unique restriction sites (XorII, BstEII, and BglII, at positions 23,033, 22,865, and 22,930, respectively) between the plant expressible kanamycin gene and border D. Note that a polylinker, i.e. a synthetic DNA segment recognized by several different restriction enzymes, might be inserted into one of the three above-mentioned restriction sites or into any other unique restriction site located between borders C and D. Useful restriction sites are those not found elsewhere in the resultant pKan3a-derivative.

4.4 Variant $T_R$-based sub-Ti plasmids

pKan3a is representative of a class of sub-Ti plasmids based on BamHI fragment 2 (Fig. 2). These plasmids include the $T_L$ right border in addition to both borders of $T_R$ (C and D) but lack all $T_L$-DNA genes. Other enzymes which do not cleave $T_R$ of the octopine-type plasmid pTi15955 and which may prove useful in construction of sub-Ti plasmids which lack functional onc genes include ApaI, SmaI (ocs, part of tml), MluI, and HpaI (part of ocs). (The $T_L$ structural genes or open reading frames (ORFs) listed parenthetically are included on the fragment generated by the preceeding enzymes.) BglI (ocs) normally cuts $T_R$-DNA but does not cut $T_R$derivatives which are deleted between the ClaI sites at positions 18,890 and 20,128. Those skilled in the art will note that the kan sequence contains a BglI site and that there are HindIII sites in pBR322 and the pUC-series plasmids. Judicious use of partial BglI digestion conditions, well understood in the art, will be needed when constructing sub-Ti plasmids based on the selectable marker constructions described herein.

Other enzymes such as AatII and ClaI may be utilized for construction of pTi15955 $T_R$-DNA-based sub-Ti plasmids which do not include border B. In particular, when grown in an appropriate methylating host, e.g. E. coli K802 (W. B. Wood (1966) J. Mol. Biol. 16:118), homogenotized T-DNA derivatives of pRK290Kan-1, pVic1, and pVic2 have no unmethylated cleavable ClaI site within $T_R$, but do have a cleavable ClaI site between border C and border B that can be used by those of ordinary skill in the art to remove border B.

In order to reduce the size of the sub-Ti plasmids discussed herein, smaller vectors may be substituted for pRK290. Plasmids that can be maintained in Agrobacterium include, but are not limited to, those described by R. C. Tait et al. (1982) Gene 20:39-49, J. Leemans et al. (1982) Gene 19:361-364, J. Hille and R. Schilperoort (1981) Plasmid 6:360-362, and G. Ditta et al. (1985) Plasmid 13:149-153.

4.5 Biological activity of pKan3a

Plant cells transformed A. tumefaciens strains harboring pKan3a were resistant to the affect of kanamycin and its analogs while controls were not. Tomato calli transformed by LBA4404 (pKan3a) were resistant to 20 mg/l of G418 (LBA4404: see G. Ooms et al. (1981) Gene 14:33-50). Sunflower hypocotyl-derived calli transformed by Bo542 (pKan3a) were resistant to 25mg/l G418 (Bo542: see E. E. Hood et al. - (1984) Biotechnol. 2:702-709). Shoots generated by transforming tobacco leaf disks (see Background) with LBA4404 (pKan3a) were resistant to 300mg/l kanamycin while control tissues died when grown of 100mg/l kanamycin.

Example 5

This Example teaches insertion of a maize zein gene into pKan3a and subsequent expression of that gene in sunflower tissues.

A zein gene encoding a 19 kd zein protein has previously been cloned into plasmid pBR322. (K. Pederson et al. (1982) Cell 29:1015-1025). A unique HindIII restriction site is present in pBR322 sequences just beyond the 3'-end of the zein gene. pKan3a does not have a HindIII site. Sticky-ends of pKan3a DNA digested with BstEII are converted to blunt-end by incubation with the Klenow fragment of E. coli DNA polymerase I. After the resultant blunt-ended DNA is mixed with and ligated to HinIII linker, it is digested with HinIII. The zein gene-containing plasmid is digested with the enzyme HindIII and the entire linearized plasmid is mixed with and ligated to the linearized, HindIII-ended, pKan3a. The recombinant plasmid derived from the insertion of the linearized zein-pBR322 plasmid into pKan3a is named pKan3a-zein. pKan3a-zein is transformed into E. coli and subsequently transferred to an A. tumefaciens helper strain, e.g. LBA4404 by the triparental mating methods. LBA4404 (pKan3a-zein) is used to inoculate plant tissue, e.g. sunflower leaf disks. After a period of growth on a medium containing kanamycin or an analog thereof, transformed plant cells are multiplied. RNA is extracted from the resultant tissue and the presence of zein mRNA is demonstrated by methods known to the art, e.g. S1 nuclease protection analysis. Furthermore, the proteins are also extracted from the multiplied tissue and the presence of zein protein is shown by specific assay. Note that transcription and translation of the zein gene remains under the control of its own eukaryotic promoter.

**Claims**

1. A DNA molecule comprising
(A) plant DNA, and
(B) a modified T-DNA,
wherein the T-DNA is inserted within the plant DNA, and wherein the T-DNA comprises
(a) an extraneous coding DNA,
(b) a foreign structural gene,
(c) a T-DNA promoter,
(d) a T-DNA polyadenylation site, and
(e) octopine-type T-DNA borders C and D,
wherein
(f) the promoter and the polyadenylation site are derived from different T-DNA genes,
(g) the promoter, the structural gene, and the polyadenylation site are in such position and orientation with respect to each other that transcription of the structural gene in a plant cell is under control of the promoter and the polyadenylation site,
(h) the promoter/structural gene/polyadenylation site combination, the extraneous coding DNA and borders C and D are positioned with respect to each other either in the arrangement "border C-combination-coding DNA-border D" or in the arrangement "border C-coding DNA-combination-border D",
(i) the T-DNA is lacking octopine-type T-DNA border A, and
(j) the T-DNA molecule is lacking all octopine-type $T_L$-DNA onc genes.
2. A DNA according to claim 1, wherein the promoter is an ORF24 promoter.
3. A DNA according to claim 1 or claim 2, wherein the polyadenylation site is an ORF25 or an ORF26 polyadenylation site.

4. A DNA according to any of claims 1 to 3, wherein the structural gene encodes neomycin phosphotransferase.

5. A DNA according to claim 4, wherein the DNA comprises the T-DNA sequence of pUC18-kan$^r$ or pKan3a.

6. A DNA according to claim 3, wherein the DNA is flanked by plant DNA.

7. A DNA according to claim 6, wherein the DNA is contained by a plant cell, a plant tissue, a plant, or a plant seed.

8. A DNA molecule comprising

(a) a foreign structural gene,

(b) a T-DNA promoter,

(c) a T-DNA polyadenylation site,

(d) octopine-type T-DNA borders C and D,

(e) a replicon permitting replication in Agrobacterium, and

(f) an extraneous coding DNA,

wherein

(g) the promoter and the polyadenylation site are derived from different T-DNA genes,

(h) the promoter, the structural gene, and the polyadenylation site are in such position and orientation with respect to each other that transcription of the structural gene in a plant cell is under control of the promoter and the polyadenylation site,

(i) the promoter/structural gene/polyadenylation site combination, the extraneous coding DNA and borders C and D are positioned with respect to each other either in the arrangement "border C-combination-coding DNA-border D" or in the arrangement "border C-coding DNA-combination-border D",

(j) the DNA molecule is lacking octopine-type T-DNA border A, and

(k) the DNA molecule is lacking all octopine-type T$_L$-DNA onc genes.

9. A DNA according to claim 8, wherein the promoter is an ORF24 promoter.

10. A DNA according to claim 8 or claim 9, wherein the polyadenylation site is an ORF25 or an ORF26 polyadenylation site.

11. A DNA according to any of claims 8 to 10, wherein the structural gene encodes neomycin phosphotransferase.

12. A DNA according to claim 11, wherein the DNA is derived from pUC18-kan $^r$ or pKan3A.

13. A DNA according to claim 9, wherein the DNA is contained in a bacterium.

14. A DNA molecule comprising

(a) a structural gene encoding neomycin phosphotransferase,

(b) an ORF24 promoter, and

(c) an ORF25 or ORF24 polyadenylation site, wherein the promoter, the structural gene, and the polyadenylation site are in such position and orientation with respect to each other that transcription of the structural gene in a plant cell is under control of the promoter and the polyadenylation site.

15. A DNA according to claim 14, wherein the DNA is essentially identical to the promoter/structural gene/polyadenylation site combination of pKan2, pUC18-kan$^r$, or pKan3a.

16. A DNA according to claim 15, wherein the DNA is pKan2, pUC18-kan$^r$, or pKan3a.

17. A method of modifying a DNA molecule comprising the step of ligating a DNA segment bearing both ORF25 and ORF26 polyadenylation sites to the 3'-end of a DNA segment bearing a structural gene, whereby in a plant cell either the ORF25 polyadenylation site or the ORF26 polyadenylation site determines the 3'-end of a transcript encoding the structural gene.

18. A method according to claim 17, wherein the polyadenylation site-bearing DNA segment is or is derived from an octopine-type T-DNA segment between HincII sites at positions 21,727 and 22,440.

19. A method of transforming a plant cell comprising the step of placing plant cell in contact with an Agrobacterium cell harboring the DNA molecule of any of claim 8 to 12.

20. A plant, plant tissue or plant seed comprising cells produced by the method of claim 19.

21. A method of preparing a DNA molecule comprising

(a) a foreign structural gene,

(b) a T-DNA promoter,

(c) a T-DNA polyadenylation site,

(d) octopine-type T-DNA borders C and D,

(e) a replicon permitting replication in Agrobacterium, and

(f) an extraneous coding DNA,

wherein

(g) the promoter and the polyadenylation site are derived from different T-DNA genes,

(h) the DNA molecule is lacking octopine-type T-DNA border A, and

(i) the DNA molecule is lacking all octopine type $T_L$-DNA <u>onc</u> genes,

said method including the step of arranging the promoter, structural gene polyadenylation site in such position and orientation with respect to each other that transcription of the structural gene in a plant cell is under control of the promoter and the polyadenylation site, and positioning the promoter/structural gene/polyadenylation site combination, the extraneous coding DNA and borders C and D with respect to each other either in the arrangement "border C-combination-coding DNA-border D' or in the arrangement "border C-coding DNA-combination-border D".

Figure 1

Figure 2

Kilo Base Pairs

# PARTIAL EUROPEAN SEARCH REPORT
which under Rule 45 of the European Patent Convention
shall be considered, for the purposes of subsequent
proceedings, as the European search report

European Patent Office

## DOCUMENTS CONSIDERED TO BE RELEVANT

EP 86307595.8

| Category | Citation of document with indication, where appropriate, of relevant passages | Relevant to claim | CLASSIFICATION OF THE APPLICATION (Int. Cl.4) |
|---|---|---|---|
| X | EP - A1 - 0 145 338 (AGRIGENETICS RESEARCH ASSOCIATES LIMITED) <br><br> * Claims 1,2,26,27,57,58,84 * | 1-3,7-10,14,20,21 | C 12 N 15/00 <br> C 07 H 21/04 <br> C 12 P 19/34 <br> A 01 H 1/00 |
| D,A | EP - A2 - 0 126 546 (AGRIGENETICS RESEARCH ASSOCIATES LIMITED) <br><br> * Claims 1,9 * | 1,8,21 | |
| A | EP - A1 - 0 122 791 (AGRIGENETICS RESEARCH ASSOCIATES LIMITED) <br><br> * Claims 1,10 * | 1,8,21 | |
| D,A | THE EMBO JOURNAL, vol. 4, no. 2, 1985 (Oxford, Washington DC) <br><br> G. AN et al. "New cloning vehicles for transformation of higher plants" pages 277-284 <br><br> * Totality * | 1,4,8,11 | |

TECHNICAL FIELDS SEARCHED (Int. Cl.4)

C 12 N
C 07 H
C 12 P
A 01 H

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-19,21

Claims searched incompletely: 20

Claims not searched: —

Reason for the limitation of the search:

(Article 53b) EPC (plant seed))

| Place of search | Date of completion of the search | Examiner |
|---|---|---|
| VIENNA | 09-01-1987 | WOLF |

EPO Form 1505.1 03 82

| Category | Citation of document with indication, where appropriate, of relevant passages | Relevant to claim | CLASSIFICATION OF THE APPLICATION (Int. Cl.4) |
|---|---|---|---|
| **DOCUMENTS CONSIDERED TO BE RELEVANT** | | | |
| P,A | WO - A1 - 86/03 516 (BIOTECHNICA INTERNATIONAL, INC.) <br><br> * Abstract; claims 1-3 * <br> -- | 1,8 | |
| A | WO - A1 - 84/02 913 (MONSANTO COMPANY) <br><br> * Claims 1-4 * <br> -- | 1,8 | |
| A | EP - A1 - 0 116 718 (MAX-PLANCK-GESELLSCHAFT ZUR FÖRDERUNG DER WISSENSCHAFTEN E.V.) <br><br> * Claims 1,9-11 * <br> ---- | 1,8 | TECHNICAL FIELDS SEARCHED (Int. Cl 4) |